# EUROPÄISCHE PATENTSCHRIFT

(11) **EP 0 470 116 B1**
(45) Veröffentlichungstag und Bekanntmachung des Hinweises auf die Patenterteilung: **14.12.1994**
(21) Anmeldenummer: 90906211.9
(22) Anmeldetag: 23.04.1990
(51) Int. Cl.: A61L 11/00, A61L 2/04, A61L 2/20

(54) **VERFAHREN ZUR STERILISATION VON MEDIZINALABFÄLLEN**
PROCESS FOR THE STERILIZATION OF MEDICAL WASTE
PROCEDE DE STERILISATION DE DECHETS MEDICAUX

(30) Priorität: 24.04.1989 DE 3913472
(43) Veröffentlichungstag der Anmeldung: 12.02.1992
(73) Patentinhaber: PILEMA S.R.L., 37135 Verona (IT)
(72) Erfinder: SCHWIENBACHER, Helmut, I-39100 Bozen (IT)
(74) Vertreter: Huber, Bernhard, Dipl.-Chem.
(86) Internationale Anmeldenummer: EP9000651
(87) Internationale Veröffentlichungsnummer: WO9012601

(56) Entgegenhaltungen:
- EP-A- 0 202 366
- CH-A- 6 565 36
- DE-A- 1 767 731

## Beschreibung

Die Erfindung betrifft ein Verfahren zur Sterilisation von toxischen Medizinalabfällen durch thermische Sterilisation mit Wasserdampf, sowie eine Vorrichtung zur Durchführung des Verfahrens.

In den letzten Jahren sind die toxischen Abfälle, die aus Spitälern oder medizinischen Zentren kommen, ein immer größeres Problem geworden, da die Möglichkeit der pathogenen Infektion von Personen, Böden, Wasser oder Luft eine große Gefahr darstellt.

Es gibt verschiedene Systeme zur chemischen Entsorgung dieser Abfälle, wie z.B. durch Ethylenoxid, Chlor, Hyprochlorit. Diese Systeme benötigen bei ihrer Anwendung jedoch ziemlich viel Zeit, schaffen weitere Umweltbelastungen und erfordern die Handhabung mit hoch-toxischen Substanzen.

Neben der chemischen Entsorgung ist eine weitere Methode in Anwendung, die auf einer Erhitzung bzw. indirekten thermischen Behandlung von Abfall in Autoklaven beruht. Dieses System hat große Nachteile auf Grund der geringen thermischen Leitfähigkeit der Luft und des Abfalles selbst. Außerdem besteht die Möglichkeit der Selbstentzündung auf Grund der im Abfall enthaltenen leicht brennbaren Substanzen, wie z.B. Papier, Watte, usw.

Aus der EP-A 287549 ist ein System der Thermobehandlung unter Verwendung von Mikrowellen und Zusatz von Wasser zur Erzeugung von Dampf, um durch direkten Kontakt zu sterilisieren, bekannt. Dieses System arbeitet schneller als die anderen; es hat sich jedoch herausgestellt, daß es in der Praxis nicht voll einsatzfähig ist, weil bei einem unterlassenen oder unzureichenden Wasserzusatz die Gefahr einer Entzündung des behandelten Mülls besteht, weil die Mikrowellen vorhandene Metalle (z.B. Nadeln von Spritzen usw.) auf über 400°C erhitzen und so Papier oder Watte im Abfall entzünden können. Dieses System ist deshalb nicht ohne Sicherheitsrisiko einsetzbar und birgt somit große Gefahren für den Betreiber.

In Anbetracht der Tatsache, daß kein sicheres und einfaches System zur Behandlung und zur Entsorgung von Medizinalabfällen angeboten wird, ist vor kurzem die Gesetzgebung in einigen Staaten, z.B. in Italien, dahingehend geändert worden, daß zur Entsorgung von Spitalmüll ausschließlich die thermische Zerstörung als einzige sichere Entsorgungsart zugelassen ist. Danach muß die Entsorgung in geeigneten Rotationsöfen mit Hochtemperaturverbrennungsanlagen durchgeführt werden, wie sie für toxische und schädliche Stoffe vorgeschrieben sind.

Die Kosten für derartige Verfahren sind jedoch äußerst hoch auf Grund der Komplexizität der Entsorgungssysteme, der hohen benötigten Energien (hohe Temperaturen) und der langen durch Wartung der Anlagen verursachten Stillstandperioden. Die Kosten dieser Entsorgungssysteme liegen bei mindestens 900 italienische Lire/kg. Für 1 Million Spitalbetten, die etwa für ein Land wie Italien mit 50 Millionen Einwohnern benötigt werden, betragen die täglichen Entsorgungskosten also mindestens 900 Millionen Lire/Tag, wenn man davon ausgeht, daß pro Bett und Tag ca. 1 kg Abfall erzeugt wird.

Außerdem besteht bei diesen Systemen der große Nachteil, daß der Müll im toxischen Zustand außerhalb des Spitalbereichs transportiert werden muß. Der toxische Spitalmüll muß zudem innerhalb einer gesetzlich vorgeschriebenen kurzen Frist, z.B. innerhalb von 48 Stunden, entsorgt werden; bei unvorhergesehenen Pannen und einem Stillstand dieser Verbrennungsöfen kann es dabei zu schwierigen Situationen kommen, weil die gesetzlich vorgeschriebene Zeit dann nicht eingehalten werden kann und zivil- und strafrechtliche Konsequenzen für die Verantwortlichen können die Folge sein.

Aufgabe der vorliegenden Erfindung war es deshalb, ein Entsorgungssystem für toxische Medizinalabfälle, insbesondere aus Spitälern, bereitzustellen, das die vorstehend genannten Nachteile vermeidet und das eine problemlose, insbesondere sichere, kostengünstige, schnelle und einfache Entsorgung ermöglicht. Diese Aufgabe wird mit der vorliegenden Erfindung gelöst.

Gegenstand der vorliegenden Erfindung ist ein Verfahren zur Sterilisation von toxischen Medizinalabfällen durch thermische Sterilisation mit Wasserdampf, das dadurch gekennzeichnet ist, daß man die Abfälle in einer Atmosphäre die einen gegenüber Luft verringerten Sauerstoffgehalt aufweist, in direkten Kontakt mit Wasserdampf bringt.

Bevorzugte Ausführungsformen dieses Verfahrens sind Gegenstand der Ansprüche 2 bis 26.

Das erfindungsgemäße Verfahren kann unter vollständigem Sauerstoffausschluß durchgeführt werden; vorzugsweise arbeitet man mit einem teilweisen Sauerstoffausschluß, und insbesondere mit einer Atmosphäre, die weniger als 15 Volumen-% Sauerstoff, vorzugsweise weniger als10 Volumen-% Sauerstoff, und insbesondere 8 oder weniger Volumen-% Sauerstoff enthält.

Die Senkung des Sauerstoffgehaltes wird zweckmäßigerweise durch teilweisen oder vollständigen Ersatz der den Sauerstoff enthaltenden Atmosphäre (Luft) durch ein inertes Gas (z.B. Stickstoff) durchgeführt, und insbesondere durch eine Spülung der Verfahrensapparatur mit diesem inerten Gas.

In einer anderen bevorzugten Ausführungsform erfolgt die Sauerstoffreduzierung über ein dafür geeignetes, handelsübliches Molekularsieb, z.B. durch Inkontaktbringen oder Durchleiten von Luft oder der Atmosphäre mit bzw. durch ein solches Molekularsieb.

Zweckmäßigerweise wird der Dampf für die Dampfbehandlung durch Erhitzen von Wasser mittels einer Mikrowellen-Heizung erzeugt; in einer bevorzugten Ausführungsform wird zu diesem Zweck das Wasser und der Abfall in geeigneten Behältern im gleichen Raum einer Mikrowellen-Bestrahlung unterworfen.

Das Wasser wird vorzugsweise in einer Menge von 5 bis 20 Gew.-%, bezogen auf das Gewicht des Medizinalabfalls (entsprechend 5 bis 20 l Wasser/100 kg Abfall) eingesetzt.

In einer bevorzugten Ausführungsform des erfindungsgemäßen Verfahrens wird zusätzlich zur thermischen Sterilisation durch Dampfbehandlung eine chemische Sterilisation durchgeführt, wie z.B. in erster Linie eine Sterilisation mit Ozon; eine solche zusätzliche Sterilisation kann durch Inkontaktbringen des zu sterilisierenden Medizinalabfalls mit Ozon oder einer ozonisierenden Substanz durchgeführt werden. Die Dampfbehandlung und die Behandlung mit Ozon können dabei in beliebiger Reihenfolge gleichzeitig, hintereinander oder auch sich teilweise überschneidend erfolgen. In einer Ausführungsform kann z.B. zu Beginn des Verfahrens (der Dampfbehandlung) Ozon in einer ausreichenden Menge zugesetzt werden und/oder es wird während der gesamten Verfahrensdauer laufend Ozon zugeführt, z.B. durch Eindüsen von Ozongas oder durch Einsprühen von ozonhaltigem Wasser. Es ist z.B. auch möglich, der Dampfbehandlung eine Sterilisationsstufe mit Ozon voranzustellen, oder eine oder mehrere Dampfbehandlungen und Behandlungen mit Ozon alternierend und in beliebiger Reihenfolge durchzuführen. Zweckmäßigerweise sollte das Sterilisationsverfahren aber zumindest während eines Teiles der Dauer der Dampfbehandlung in Gegenwart von Ozon durchgeführt werden, und in erster Linie wird die thermische Dampfbehandlung während der gesamten Dauer in einer ozonhaltigen Atmosphäre durchgeführt, d.h. der zu sterilisierende Abfall ist während der gesamten Zeit in Kontakt mit Ozon. Zur Aufrechterhaltung einer solchen ozonhaltigen Atmosphäre während der gesamten Verfahrensdauer kann das Ozon zumindest vor Beginn der Erhitzungsphase in einer ausreichenden Mengen zugeführt werden, insbesondere wird aber während der gesamten Verfahrensdauer Ozon zugeführt.

Das Ozon kann in Form eines ozonhaltigen Gasstromes, z.B. mit einem Ozongehalt von 2 bis 20 Volumen-%, oder in Form einer vorzugsweise gesättigten wäßrigen Lösung zugeführt und in Kontakt mit dem Abfall gebracht werden. Die Herstellung des ozonhaltigen Gasstromes bzw. der wäßrigen Lösung kann auf eine an sich bekannte Weise, z.B. durch elektrische Entladung oder elektrochemisch mit einem üblichen Ozongenerator und gegebenenfalls Auflösung in Wasser erfolgen. Die Zuführung der wäßrigen Ozonlösung erfolgt zweckmäßigerweise durch Versprühen der Lösung in den Abfallbehälter und/oder in den Reaktionsraum.

Die Figur 3 zeigt schematisch eine Vorrichtung, mit der gleichzeitig oder wahlweise Ozon über die Leitung (35) und Wasser oder mit Ozon gesättigtes Wasser über die Leitung (36) entnommen werden können: Der Ozongeneratir (37) ist mit einem Mischer (38), in den über die Leitung (39) Wasser eingeleitet werden kann, über das Rohr (40) verbunden. Über Vorrichtungen zur Messung des Ozongehaltes und über in den Leitungen (35) und (36) befindliche Ventile kann die Mischapparatur, und die Entnahme von Ozon und/oder Wasser und/oder von mit Ozon gesättigtem Wasser gesteuert werden.

Die gesättigte wäßrige Ozonlösung wird vorzugsweise in einer Menge von 5 bis 20 Gew.-%, entsprechend 5 bis 20 l wäßriger Lösung/100 kg Abfall, eingesetzt; die Dauer der Ozoneinführung beträgt nach Erreichen der Siedetemperatur (in der Regel 90 bis 120°C) vorzugsweise weitere 5 bis 15 Minuten.

Auch die Stufe der Sterilisation mit Ozon wird, wie die Dampfbehandlungsstufe, zweckmäßigerweise in einer Atmosphäre durchgeführt, die einen gegenüber Luft verringerten Sauerstoffgehalt aufweist. Vorzugsweise besitzt diese Atmosphäre auch bei einer getrennt durchgeführten Ozonbehandlungsstufe, die sich also nicht mit der Dampfbehandlung überschneidet oder mit ihr zusammenfällt, den gleichen Sauerstoffgehalt wie die Atmosphäre der Dampfbehandlung.

Im erfindungsgemäßen Verfahren kann die Stufe der Dampfbehandlung und/oder Behandlung mit Ozon bei Überdruck oder Normaldruck durchgeführt werden; in Kombination mit Ozon wird vorzugsweise bei Normaldruck gearbeitet. Ohne Ozoneinsatz kann es zweckmäßig sein, durch Druckerhöhung die Sterilisationstemperatur zu erhöhen. Obwohl es auch möglich ist, unter reduziertem Druck, also unter Vakuum, zu arbeiten, wodurch auch eine Verringerung des Sauerstoffgehaltes erreicht wird und bei der Zufuhr der wäßrigen Ozonlösung unter Abbau des Unterdruckes eine innigere Vermischung (besseres Eindringen) mit dem Medizinalabfall erreicht werden kann, ist es wenig zweckmäßig, den reduzierten Druck auch während der Erhitzungsphase beizubehalten, weil dadurch die Temperatur des Dampfes verringert wird. In einer Ausführungsform des erfindungsgemäßen Verfahrens arbeitet man deshalb unter teilweisem Vakuum, d.h. bis zum Beginn der Erhitzungsphase unter reduziertem Druck, z.B. bis herab zu einem Druck von 10³ Pa (0,01 at), und erhöht dann den Druck vor dem Einschalten der Mikrowellen-Heizung auf Normaldruck oder auch erhöhten Druck. Z.B. wird zur Reduktion des Sauerstoffs der Reaktionsraum, in dem sich der Abfall oder der Abfallbehälter befindet, zuerst mit Stickstoff gespült und dann evakuiert, oder zuerst evaluiert und dann mit Inertgas unter Unterdruck gespült, wobei man ein Unterdruck-Halteventil verwendet, und unter Beibehaltung des Vakuums wird eine gesättigte wäßrige Ozonlösung in den Abfallbehälter und/oder den Reaktionsraum versprüht; danach wird vor dem Einschalten der Mikrowellen-Heizung durch Öffnen eines Ventils wieder Normaldruck hergestellt oder auch ein Überdruck angelegt. Durch ein Arbeiten mit Überdruck kann die Temperatur des Wasserdampfes und damit die Effizienz der Sterilisation erhöht werden; ein Arbeiten bei Überdruck erfordert aber besondere Maßnahmen, wie z.B. eine Druckkammer. Ein Arbeiten mit Überdruck bzw. bei Temperaturen um oder oberhalb von 120°C, z.B. bei 120 bis 125°C, ist insbesondere dann zweckmäßig, wenn nur eine thermische Sterilisation mit Wasserdampf erfolgt, also ohne Ozon-Sterilisation.

Als Mikrowellen-Heizung können übliche Heizaggregate, wie sie z.B. in Mikrowellen-Herden verwendet werden (Magnetrons), dienen. Das Heizsystem ist so angeordnet, daß die erzeugten Mikrowellen jeden Teil des Reaktionsraumes erfassen. Der Reaktionsraum ist vorzugsweise eine Kammer, von der mindestens eine neben und/oder unterhalb des Abfallbehälters befindliche Wand, z.B. die untere Wand (Boden) und/oder die Seitenwände ein Mikrowellen-Heizsystem (Magnetron) enthält. Vorzugsweise ist mindestens die obere Begrenzungswand (Deckel) der Kammer mit einer Vorrichtung zum Kühlen ausgestaltet, und ist zur Durchströmung mit einem Kühlmedium z.B. doppelwandig ausgebildet.

Als Behälter für den Medizinalabfall können dafür übliche Behälter eingesetzt werden, vorzugsweise Abfallkartons aus Pappe pder einem ähnlichen Material, die als Faltschachteln (Faltkartons) handelsüblich erhältlich sind. Vorzugsweise werden die Kartons mit temperaturbeständigen PE-Innenbeuteln oder wasserfesten Polyethylen(PE)-Beschichtungen verwendet. Vorzugsweise ist der Karton an seiner Oberseite mit einem Deckel und einer Zuleitungsöffnung versehen, durch die z.B. ein beweglicher Rüssel, der im Inneren mehrere Zuleitungsschläuche enthält, hindurchgeführt werden kann. Die Behälter besitzen in der Regel ein Fassungsvermögen von 30 bis 80 l, insbesondere von ca. 40 l, zur Aufnahme von ca. 7 bis 12 kg Medizinalabfall.

Die Anordnung und Abmessungen der einzelnen Apparateteile (Größe der Abfallbehälter, Austrittsöffnung am Abfallbehälter, Form und Größe der Reaktionskanmer, Anordnung der Mikrowellen-Heizung, usw.), die Heizleistung, die Reaktionsdauer, die Wassermenge und die Menge an zugeführtem Ozon richten sich insbesondere nach der Menge und Art des zu sterilisierenden Abfalls und werden vorzugsweise so ausgewählt, daß für die gesamte Verfahrensdauer eine ausreichende Menge an Wasserdampf und Ozon vorhanden sind.

In einer bevorzugten Ausführungsform des erfindungsgemäßen Verfahrens werden ein oder mehrere der mit Medizinalabfall gefüllten Behälter, vorzugsweise vier Stück, in eine den Reaktionsraum bildende verschließbare Kammer gegeben, die mit einem Brüdenrohr für den Abzug der sich bildenden Wasserdämpfe versehen ist. Nach Verschließen der die Abfallbehälter enthaltenden Kammer wird durch eines der Zuleitungsrohre im Innern des in den Abfallbehälter eingeführten fahrbaren Rüssels und durch mehrere am unteren Teil der Kammer angebrachte Rohre Stickstoff eingeleitet, bis der Sauerstoffgehalt der Luft unter den gewünschten Wert, vorzugsweise unter 15 Volumen-%, und insbesondere unter 10 Volumen-%, abgesunken ist. Durch die zentrale Öffnung werden nun über eine Sprühdüse 0,2 bis 3 l, vorzugsweise 1 bis 2 l/7 bis 12 kg Medizinalabfall (entsprechend ca. 5 bis 20 % des Gewichtes des vorhandenen Abfalls, das sind 5 bis 20 l/100 kg Abfall) Wasser oder ozongesättigtes Wasser in den Abfallbehälter eingebracht. Nun wird die Mikrowellen-Heizung eingeschaltet. Die Mikrowellen-Heizung besitzt vorzugsweise eine Leistung, die so ausgelegt ist, daß das dazugegebene Wasser in ca. 5 Minuten zum Kochpunkt erhitzt wird (ca. 1 bis 3 KW/Behälter, bei 4 Behältern also z.B. eine Leistung von 4 bis 12 KW). Durch Erhitzung des Wasser und die damit verbundene Verminderung der Löslichkeit von Ozon wird die Bildung einer Ozonwolke begünstigt, und die desinfizierende und sterilisierende Wirkung von Ozon durch die Gegenwart von Feuchtigkeit stark erhöht. Parallel dazu tritt die sterilisierende Wirkung des Wasserdampfes unter hoher Temperatur in Kraft.

Durch die Mikrowellen-Heizung wird die Temperatur bis zum Siedepunkt des Wassers erhöht. In der Regel sind Sterilisationszeiten von 5 bis 15 Minuten ausreichend, um eine 100 %ige Garantie der Zerstörung aller pathogenen Keime zu erhalten.

Durch die erhöhte Temperatur (ca. 90 bis 120°C) wird außerdem eine Deformierung der vorhandenen Plastikteile erreicht, wodurch eine eventuelle Wiederverwendung von Medizinalabfallteilen (z.B. Spritzen) ausgeschlossen werden kann.

Zweckmäßigerweise wird auch während der Erhitzungsphase Ozon durch einen starken Sprühstrahl weiter zugegeben, wodurch eine ausreichende Ozonatmosphäre während der gesamten Dauer der thermischen Behandlung mit Wasserdampf sichergestellt wird.

Es ist auch zweckmäßig, den Deckel des Abfallbehälters zu kühlen, z.B. mit einem kalten Stickstoffstrom. Dadurch wird eine Kondensation des Wasserdampfes am Deckel und ein Zurücktropfen in den Abfall erreicht; freigesetztes Ozon wird in dem zurückfließenden Kondensat gelöst und kann so erneut auf den Abfall einwirken.

Obwohl der Zerfall von Ozon in der Wärme sehr rasch erfolgt, genügen für das erfindungsgemäße Verfahren überraschenderweise bereits sehr geringe Mengen, um die Sterilisation rasch und vollständig durchzuführen, und zwar in der Regel Konzentrationen von 0,01 bis 1,0 Volumen-% Ozon im Gasraum. Durch überschüssiges Ozon wird außerdem noch eine Sterilisation des Reaktionsraumes und der austretenden Brüden bewirkt.

Zweckmäßig ist es, die Zufuhr von Ozon, vorzugsweise in Form von Gas oder einer wäßrigen Ozonlösung, auch während der Abkühlphase fortzusetzen; dadurch kann auf Grund des hohen Feuchtigkeitsgehaltes im Abfallbehälter eine optimale Sterilisationswirkung erreicht werden. Dies gilt auch für die Zerstörung von gegebenenfalls vorhandenen übelriechenden Substanzen auf Amin- oder Sulfidbasis.

Die Wärmebehandlung (Erhitzungsphase), die vorzugsweise ca. 5 bis 15 Minuten beträgt, wird durch Abschaltung der Heizung beendet. In der sich daran anschließenden Abkühlphase wird die Abkühlung zweckmäßigerweise durch Zufuhr eines Kühlmittels unterstützt, z.B. durch Spülung mit einem kalten Stickstoff-Strom (vorzugsweise von Raumtemperatur); vorzugsweise wird die Oberseite des Reaktionsraumes, z.B. der Deckel der Mikrowellen-Kammer, und/oder der Deckel des Abfallbehälters gekühlt. Eine Kühlung des Deckels des Abfallbehälters ist z.B. über die im fahrbaren Rüssel befindliche Stickstoffzuleitung möglich.

Das erfindungsgemäße Verfahren kann in einer dafür geeigneten Anlage auch kontinuierlich durchgeführt werden , z.B. in einem Tunnel, einem Drehteller oder einer ähnlichen technischen Vorrichtung unter Druck oder bei Normaldruck mit oder ohne Anwendung von Ozon.

Eine bevorzugte Ausführungsform des erfindungsgemäßen Verfahrens ist durch die folgenden hintereinander durchgeführten Verfahrensstufen gekennzeichnet:
a) Einbringen eines oder mehrerer Abfallbehälter, in denen sich der zu sterilisierende Abfall befindet, in eine mit einer Mikrowellen-Heizung und einem Abzugsrohr versehenen Kammer,
b) Absenkung des Sauerstoffgehaltes im Inneren der Kammer auf weniger als 15 Volumen-%,
c) Einsprühen von Wasser oder einer wäßrigen Ozonlösung in den Abfallbehälter und/oder in das Innere der Kammer,
d) Einschalten der Mikrowellen-Heizung und Erhitzen auf eine zur Erzeugung von Wasserdampf ausreichenden Temperatur mit oder ohne Zufuhr von Ozon und
e) Abkühlung.

Um jeden Unfall ausschließen zu können und die absolute Effizienz der Behandlung zu garantieren, ist das Behandlungssystem vorzugsweise mit verschiedenen, meist doppelwirkenden Sicherheitseinrichtungen ausgerüstet: Es ist vorgesehen, daß die Heizung nicht startet, wenn kein Wasser oder eine wäßrige Ozonlösung zugesetzt wurde. Die Anlage wird im Inneren durch Pressostaten überwacht. Trotz der Reduzierung von Sauerstoff wird ein CO₂-Spürgerät mit Sprinklersystem vorgesehen, um eventuelle Brände sofort bekämpfen zu können.

Um eine Umweltbelastung auszuschalten oder auf ein Minimum zu reduzieren, werden die austretenden Dämpfe kondensiert und danach chemisch, z.B. durch Ozon und/oder, unabhängig davon, durch UV-Bestrahlung sterilisiert; dadurch sind sowohl Luft als auch das austretende Wasser vollkommen ungefährlich.

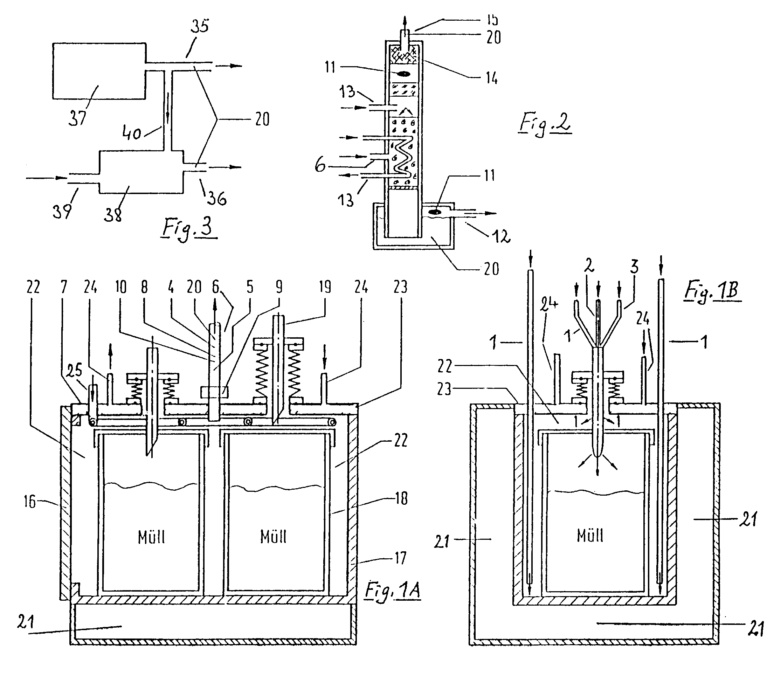

Die Figur 1 zeigt eine schematische Darstellung einer zur Durchführung des erfindungsgemäßen Verfahrens geeigneten Vorrichtung im Längsschnitt (Figur 1A) und Querschnitt (Figur 1B):
Eine als Druckkammer ausgebildete Kammer (17) zur Aufnahme und Sterilisation von in einem Behälter befindlichen Medizinalabfall ist an ihrem oberen Teil mit einem Brüdenrohr (6) zur Dampfableitung versehen; im Boden und an den Seiten der Kammer ist ein Mikrowellen-Heizsystem (21), z.B. Magnetrone, integriert; Einrichtungen (1) zur Einleitung eines Inertgases zur Reduzierung des Sauerstoffgehaltes im Innern der Kammer (17) sind vorhanden. Der Deckel der Kammer (23) ist doppelwandig ausgebildet, und mit Zu- und Ableitungen (24) für ein Kühlmittel, z.B. Stickstoffgas, versehen.

Im Innern der Kammer befinden sich zwei Abfallbehälter (18), die den zu sterilisierenden Medizinalabfall enthalten. Dieser Behälter (18) kann durch geeignete verschließbare Öffnungen (Tür, Klappe) (16) in die Kammer eingebracht und dieser entnommen werden. Durch das Brüdenrohr (6) wird in eine zentrale Öffnung im Deckel des Abfallbehälters (18) ein als Füllstößel ausgebildeter fahrbarer Rüssel (19) eingeführt, der im Innern mehrere Zuleitungen (Schläuche) für das ozonhaltige Gas (3) für Wasser oder eine gesättigte wäßrige Ozonlösung (2) und/ oder für ein inertes gasförmiges Kühlmittel, z.B. Stickstoff (1), enthält. Am Brüdenrohr (6) sind geeignete Einrichtungen zur Messung und Steuerung der Temperatur (8), des Druckes (9), des Sauerstoffgehaltes (4), des Feuchtigkeitsgrades (10) und zur CO₂-Überwachung (5) vorgesehen. Am Kammerdeckel ist im Inneren der Kammer (17) eine Sprinkleranlage (7) mit einer durch den Kammerdeckel führenden Zuleitung (25) vorgesehen.

In einer alternativen, nicht gezeigten Ausführungsform ist die Kammer (17) zusätzlich mit Zuleitungen für Ozon und/oder Kühlmittel in den Kammerraum (22) und/oder den Abfallbehälter (18) ausgestattet, die z.B. durch den Deckel und/oder die Seitenwände der Kammer (17) geführt werden. Die Zuleitungen für Wasser oder die wäßrige Ozonlösung sind an ihrem Ende vorzugsweise mit Sprühdüsen versehen.

Wenn die Kammer (17) zur Aufnahme von mehreren Abfallbehältern, z.B. von vier Behältern, vorgesehen ist, ist es zweckmäßig, die Kammer auch mit mehreren Brüdenrohren oder zusätzlichen Öffnungen zur Einführung mehrerer fahrbarer Rüssel und/oder Zuleitungen für Wasser und/oder Ozon und/oder Kühlmittel zu versehen.

Gegenstand der Erfindung ist deshalb auch eine Vorrichtung gemäß Anspruch 27 zur Durchführung des erfindungsgemäßen Verfahrens zur Sterilisation von Medizinalabfall, bestehend aus einer Kammer (17), ein Brüdenrohr (6), die ein Mikrowellen-Heizsystem (21) zur Beheizung des Kammerinneren (22) und eine Einrichtung (1) zur Einführung eines Inertgases und/oder ein Molekularsieb zur Reduzierung des Sauerstoffgehaltes der Kammeratmosphäre aufweist.

Zweckmäßige Ausführungen davon sind Gegenstand der Ansprüche 28 bis 32. unter Verwendung eines Molekularsiebes erfolgt die Reduzierung des Sauerstoffgehaltes durch Inkontaktbringen der Kammeratmosphäre mit einem im Inneren (22) der Kammer (17) angebrachten Molekularsieb, oder durch Durchleiten der Kammeratmosphäre durch ein Molekularsieb über ein mit einer Pumpe versehenes Leitungssystem im Kreislauf; oder durch Durchleiten von sauerstoffarmer Luft (erzeugt durch Absorption von Sauerstoff mit externen Molekularsieben).

Vorzugsweise ist der Deckel (23) der Kammer zur Durchströmung mit einem Kühlmittel doppelwandig ausgebildet und mit Zuund Ableitungen (24) für das Kühlmittel versehen.

In einer weiteren zweckmäßigen Ausführungsform ist die Kammer (17) mit Zuleitungen für Wasser oder eine wäßrige Ozonlösung, für Ozon und/oder ein inertes, gasförmiges Kühlmittel, z.B. Stickstoff, in den Kammerraum (22) und/oder in in dem Kammerraum (22) befindliche Abfallbehälter (18) ausgestattet.

Um Unfällen beim Start des erfindungsgemäßen Verfahrens und während der Durchführung des Verfahrens vorzubeugen und um eine hohe Verfahrenseffizienz zu gewährleisten, ist die Vorrichtung vorzugsweise zusätzlich mit einer oder mehreren der folgenden Einrichtungen versehen:
a) einer Einrichtung zur vorzugsweise automatischen Versprühung von Wasser oder einer wäßrigen Ozonlösung in die Kammer und/oder einen sich in der Kammer befindlichen Abfallbehälter beim Start eines Verfahrenszyklus,
b) einer automatischen, mit einer Sprinkleranlage (7) verbundenen Feuerschutzkontrolle,
c) automatischen Meß- und Steuereinrichtungen für Temperatur (8), Druck (9), Sauerstoffgehalt (4) und/oder Feuchtigkeitsgrad (10) im Innern der Kammer (22).

Um eine Umweltbelastung durch das erfindungsgemäße Verfahren auszuschalten oder zumindest auf ein Minimum zu reduzieren, wird die Vorrichtung vorzugsweise mit einer an sich bekannten Einrichtung zur direkten oder indirekten Kühlung zur Kondensation der aus dem Brüdenrohr (6) austretenden Brüden versehen, wie z.B. mit einem mit einer Kühlschlange oder einem Kühlmantel versehenen Rohr, durch das der Brüdenstrom geleitet wird. Figur 2 zeigt eine Ausführungsform einer solchen Vorrichtung. Der Kühlvorrichtung (13), durch die Kühlwasser strömt, wird vorzugsweise eine Einrichtung für eine ebenfalls an sich bekannte chemische Sterilisation, z.B. mittels Ozon (Ozonfilter 14) und/oder für eine Sterilisation durch UV-Bestrahlung der kondensierten Brüden (UV-Lampe 11) nachgeschaltet. Am Austritt der sterilen Abluft (15) und des sterilen Abwassers wird eine Vorrichtung zur Ozon-Messung (20) angebracht, das sterile Abwasser strömt durch das Rohr (12) ab.

Die Kammer mit den zusätzlichen Einrichtungen (Brüdenrohr, Zu- und Ableitungen usw.) besteht aus einem gegenüber der Sterilisationsatmosphäre (Wasserdampf, Ozon) beständigen Material, vorzugsweise aus eloxiertem Aluminium, pulverbeschichtetem Aluminium, verchromten Metallteilen und/oder Edelstahl.

Das nachfolgende Beispiel soll die Erfindung näher erläutern, ohne sie darauf zu beschränken.

### Beispiel

In eine Vorrichtung gemäß Figur 1 wird ein Abfallbehälter von 40 l Inhalt, der ca. 10 kg Spitalmüll enthält, gegeben. Ein fahrbarer Rüssel (19), der Zuleitungen für Wasser oder eine wäßrige Ozonlösung, Ozongas und Stickstoff oder Inertgas enthält, wird in die zentrale Öffnung des Abfallbehälterdeckels eingeführt. Durch eine Zuleitung des fahrbaren Rüssels und durch am Boden der Kammer befindliche Zuleitungen (1) wird Stickstoff eingeleitet, bis der Sauerstoffgehalt der Kammeratmosphäre auf einen Wert unter 15 Volumen-% gesunken ist. Über die Ozonzuleitung, die an ihrem in den Abfallbehälter eintauchenden Ende mit einer Sprühdüse versehen ist, werden ca. 2 l Wasser oder einer gesättigten wäßrigen Ozonlösung eingesprüht. Danach wird die Mikrowellen-Heizung (2,5 KW) eingeschaltet.

Durch die nunmehr stattfindende Erhitzung der wäßrigen Lösung und die damit verbundene Löslichkeitsverringerung des Ozons in Wasser bildet sich in der Kammer sehr rasch eine Ozonwolke, die unmittelbar desinfizierend und sterilisierend wirkt. Diese Wirkung wird durch Einleiten von Ozongas und durch die Gegenwart des Wasserdampfes noch stark erhöht, und parallel zur sterilisierenden Wirkung von Ozon tritt die sterilisierende Wirkung des heißen Wasserdampfes in Kraft. Nach Erreichung der Siedetemperatur des Wassers (nach ca. 5 Minuten) wird weitere 10 Minuten bei dieser Temperatur erhitzt. Während der gesamten Erhitzungsphase wird die Zufuhr von Ozon weitergeführt, um eine ausreichende Ozonatmosphäre während der gesamten Dauer sicherzustellen; dabei wird der Deckel des Abfallbehälters durch Stickstoff aus der Stickstoff-Leitung des fahrbaren Rüssels gekühlt, wodurch eine Kondensation des Wasserdampfes erreicht wird und das Kondensat sich wieder mit Ozon belädt.

Auch nach Abschaltung der Mikrowellen-Heizung (Beendigung der Erhitzungsphase) wird die Zufuhr von Ozon weiter aufrechterhalten, um eine optimale Sterilisationswirkung auf Grund des hohen Feuchtigkeitsgehaltes zu erzielen. Durch überschüssiges Ozon wird außerdem gleichzeitig eine Sterilisationswirkung auf die ausströmenden Brüden ausgeübt, und eine Zerstörung von gegebenenfalls vorhandenen unangenehm riechenden Stoffen auf Amin- oder Sulfidbasis erreicht. Nach Abschaltung der Mikrowellen-Heizung kann die Abkühlung durch Einleiten von Stickstoff durch die Zuführungen (5) im Boden der Kammer beschleunigt werden. Es wird auf Umgebungstemperatur abgekühlt und der Abfallbehälter dann aus der Kammer entnommen. Es wird eine praktisch 100 %ige Sterilisation des Medizinalabfalls (Zerstörung aller pathogenen Keime) erreicht.

Mit dem erfindungsgemäßen System ist es möglich, die Entsorgungskosten für Medizinalabfall auf mehr als die Hälfte zu reduzieren. Außerdem ist es damit möglich, den entstehenden Abfall mit hoher Effizienz bereits am Entstehungsort zu behandeln und ihn in problemlosen Abfall umzuwandeln, der mit normalem Hausabfall vergleichbar ist, gefahrlos transportiert und mit geringen Kosten auf Deponien abgelagert oder in üblichen Verbrennungsanlagen zerstört werden kann. Störfälle durch Eigenentzündung lassen sich erfindungsgemäß sicher vermeiden und der Zeitaufwand (Dauer eines Sterilisationszyklus) stark verringern. Das erfindungsgemäß eingesetzte Ozon kann leicht am Ort der Applikation erzeugt werden und läßt sich problemlos und vollständig beseitigen (z.B. durch Aktivkohle oder Katalysatoren, wobei es in unschädlichen Sauerstoff zerfällt), wodurch keine Umweltbelastung auftritt. Mit dem Verfahren und der Vorrichtung gemäß der vorliegenden Erfindung wird also ein Entsorgungssystem für Medizinalabfall bereitgestellt, das einfach, kostengünstig, sicher und rasch durchführbar ist und mit dem eine praktisch vollständige Sterilisation ohne Umweltfolgebelastungen erreicht wird. Es ist auf alle in Medizinalabfällen auftretenden Pathogene (Mikroorganismen, Viren, vegetative Formen von Keimen, Sporen, Parasiten, Pilze, usw.) anwendbar.

## Patentansprüche

1. Verfahren zur Sterilisation von toxischen Medizinalabfällen durch thermische Sterilisation mit Wasserdampf, dadurch gekennzeichnet, daß man die Abfälle in einer Atmosphäre, die einen gegenüber Luft verringerten Sauerstoffgehalt aufweist, in direkten Kontakt mit Wasserdampf bringt.

2. Verfahren nach Anspruch 1, dadurch gekennzeichnet, daß man unter teilweisem oder vollständigem Sauerstoffausschuß arbeitet.

3. Verfahren nach Anspruch 1 oder 2, dadurch gekennzeichnet, daß der Dampf durch Erhitzen von Wasser mittels einer Mikrowellenheizung erzeugt wird.

4. Verfahren nach Anspruch 3, dadurch gekennzeichnet, daß sich das Wasser und der Abfall in dem gleichen, einer Mikrowellenbestrahlung unterworfenen Raum befinden.

5. Verfahren nach einem der Ansprüche 1 bis 4, dadurch gekennzeichnet, daß zusätzlich zur thermischen Sterilisation durch Dampfbehandlung eine Sterilisation mit Ozon durchgeführt wird.

6. Verfahren nach Anspruch 5, dadurch gekennzeichnet, daß die Dampfbehandlung und die Behandlung mit Ozon in beliebiger Reihenfolge gleichzeitig, hintereinander oder sich teilweise überschneidend durchgeführt werden.

7. Verfahren nach Anspruch 5 oder 6, dadurch gekennzeichnet, daß eine oder mehrere Dampfbehandlungen und Behandlungen mit Ozon alternierend durchgeführt werden.

8. Verfahren nach einem der Ansprüche 5 bis 7, dadurch gekennzeichnet, daß das Verfahren zumindest während eines Teiles der Dauer der Dampfbehandlung in Gegenwart von Ozon durchgeführt wird.

9. Verfahren nach einem der Ansprüche 5 bis 8, dadurch gekennzeichnet, daß das Ozon als gesättigte wäßrige Ozonlösung und/oder als Gas mit dem zu sterilisierenden Abfall in Kontakt gebracht wird.

10. Verfahren nach Anspruch 9, dadurch gekennzeichnet, daß man die thermische Dampfbehandlung während der gesamten Dauer in einer ozonhaltigen Atmosphäre durchführt.

11. Verfahren nach Anspruch 10, dadurch gekennzeichnet, daß man zumindest während der Erhitzungsphase eine zur Aufrechterhaltung der Ozonatmosphäre ausreichende Menge an Ozon zuführt.

12. Verfahren nach Anspruch 10, dadurch gekennzeichnet, daß man Ozon während der gesamten Verfahrensdauer in Form einer wäßrigen Lösung versprüht.

13. Verfahren nach einem der Ansprüche 5 bis 12, dadurch gekennzeichnet, daß man die Sterilisation mit Ozon in einer Atmosphäre durchführt, die einen gegenüber Luft verringerten Sauerstoffgehalt aufweist.

14. Verfahren nach Anspruch 13, dadurch gekennzeichnet, daß man die Sterilisation mit Ozon in der gleichen Atmosphäre wie die Dampfbehandlung durchführt.

15. Verfahren nach einem der vorhergehenden Ansprüche, dadurch gekennzeichnet, daß die Atmosphäre, in der die Stufen der Dampfbehandlung und/oder der Behandlung mit Ozon durchgeführt werden, einen Sauerstoffgehalt von kleiner als 15 Volumen-% besitzt.

16. Verfahren nach Anspruch 15, dadurch gekennzeichnet, daß der Sauerstoffgehalt 10 Volumen-% oder weniger beträgt.

17. Verfahren nach einem der vorhergehenden Ansprüche, dadurch gekennzeichnet, daß man die Stufen der Dampfbehandlung und/oder Behandlung mit Ozon bei Normaldruck oder bei Überdruck durchführt.

18. Verfahren nach einem der vorhergehenden Ansprüche, gekennzeichnet durch die folgenden hintereinander durchgeführten Verfahrensschritte:
a) Einbringen einer oder mehrerer Behälter, in denen sich der zu sterilisierende Abfall befindet, in eine mit einer Mikrowellenheizung und einem Abzugsrohr versehene Kammer,
b) Absenkung des Sauerstoffgehaltes im Innern der Kammer auf weniger als 10 Volumen-%,
c) Einsprühen von Wasser oder einer wäßrigen Ozonlösung in den Abfallbehälter und/oder in das Innere der Kammer,
d) Einschalten der Mikrowellenheizung und Erhitzen auf eine zur Erzeugung von Wasserdampf ausreichende Temperatur bei Normaldruck oder Überdruck von bis 0.2 MPa (2 bar), und
e) Abkühlung.

19. Verfahren nach Anspruch 18, dadurch gekennzeichnet, daß man die Absenkung des Sauerstoffgehaltes im Verfahrensschritt b) durch Einleiten eines Inertgases, vorzugsweise Stickstoff, in den mit einer Öffnung versehenen Abfallbehälter und/oder in die Kammer und/oder durch Inkontaktbringen oder Durchleiten der Kammeratmosphäre mit bzw. durch ein Molekularsieb und/oder Einbringen von mit außenstehenden Molekularsieben erzeugter sauerstoffarmer Luft durchführt.

20. Verfahren nach Anspruch 18 oder 19, dadurch gekennzeichnet, daß man im Verfahrensschritt c) Wasser oder eine gesättigte wäßrige Ozonlösung in einer Menge von 5 bis 20 l/100 kg Abfall zuführt.

21. Verfahren nach einem der Ansprüche 18 bis 20, dadurch gekennzeichnet, daß die Kammer zumindest durch eine, vorzugsweise aber mehrere unterhalb oder seitlich des Abfallbehälters angebrachte Mikrowellenheizungen erhitzt wird.

22. Verfahren nach einem der Ansprüche 18 bis 21, dadurch gekennzeichnet, daß die Verfahrensdauer 5 bis 30 Minuten beträgt.

23. Verfahren nach einem der Ansprüche 18 bis 22, dadurch gekennzeichnet, daß man während der gesamten Erhitzungsdauer eine wäßrige Ozonlösung oder Ozongas zuführt.

24. Verfahren nach Anspruch 23, dadurch gekennzeichnet, daß man die Zuführung der wäßrigen Ozonlösung oder des Ozongases auch während der auf die Erhitzungsphase folgenden Abkühlungsphase e) fortsetzt.

25. Verfahren nach einem der Ansprüche 18 bis 24, dadurch gekennzeichnet, daß man während der Erhitzungsphase d) den Deckel des Abfallbehälters, vorzugsweise durch Spülung mit Stickstoff, kühlt.

26. Verfahren nach einem der Ansprüche 18 bis 25, dadurch gekennzeichnet, daß man während der Abkühlungsphase e) den Deckel der Kammer und/oder des Abfallbehälters, vorzugsweise durch Spülung mit Stickstoff kühlt.

27. Vorrichtung zur Durchführung des Verfahrens nach einem der Ansprüche 1 bis 26, bestehend aus einer Kammer (17), die ein Brüdenrohr (6), ein Mikrowellen-Heizsystem (21) zur Beheizung des Kammerinneren (22) und eine Einrichtung (1) zur Einführung eines Inertgases und/oder ein Molekularsieb zur Reduzierung des Sauerstoffgehaltes der Kammeratmosphäre aufweist.

28. Vorrichtung nach Anspruch 27, dadurch gekennzeichnet, daß der Deckel (23) der Kammer zur Durchströmung mit einem Kühlmittel doppelwandig ausgebildet und mit Zu- und Ableitungen (24) für das Kühlmittel versehen ist.

29. Vorrichtung nach einem der Ansprüche 27 und 28, dadurch gekennzeichnet, daß sie mit Zuleitungen für Wasser oder eine wäßrige Ozonlösung und/oder ein inertes gasförmiges Kühlmittel in den Kammerraum (22) und/oder in in dem Kammerraum (22) befindliche Abfallbehälter (18) vergehen ist.

30. Vorrichtung nach einem der Ansprüche 27 bis 29, dadurch gekennzeichnet, daß sie mit einer oder mehreren der folgenden Einrichtungen versehen ist:
a) eine Einrichtung zur Versprühung von Wasser oder einer wäßrigen Ozonlösung in die Kammer und/oder einen sich in der Kammer befindlichen Abfallbehälter beim Start eines Verfahrenszyklus,
b) einer automatischen, mit einer Sprinkleranlage (7) verbundenen Feuerschutzkontrolle (5),
c) automatischen Meß- und Steuereinrichtungen für Temperatur (8), Druck (9), Sauerstoffgehalt (4) und/oder Feuchtigkeitsgrad (10) im Innern der Kammer.

31. Vorrichtung nach einem der Ansprüche 27 bis 30, dadurch gekennzeichnet, daß sie mit einer Einrichtung zur direkten oder indirekten Kühlung zur Kondensation der aus dem Brüdenrohr (2) austretenden Brüden versehen ist.

32. Vorrichtung nach Anspruch 31, dadurch gekennzeichnet, daß sich an die Kühleinrichtung zur Kondensation der Brüden eine Einrichtung zur chemischen Sterilisation und/oder für eine Sterilisation durch UV-Bestrahlung der kondensierten Brüden anschließt.

## Claims

1. Process for the sterilization of toxic medical waste by thermal sterilization with steam, characterized by the fact that the waste is brought into direct contact with the steam in an atmosphere of reduced oxygen concentration compaired with air.

2. Process according to claim 1, characterized by an operation under partial or complete oxygen exclusion.

3. Process according to claim 1 or 2, characterized by the fact that the steam is generated by heating water by means of a microwave heater.

4. Process according to claim 3, characterized by the fact that the water and the waste are introduced into the same chamber and submitted to microwave radiation.

5. Process according to one of the claims 1 to 4, characterized by the fact that in addition to the thermal sterilization through steam treatment a sterilization with ozone is effected.

6. Process according to claim 5, characterized by the fact that the steam treatment and the ozone treatment can be carried out in an arbitrary sequence: either simultaneously, successively or partly overlapping.

7. Process according to claim 5 or 6, characterized by the fact that one or several steam treatments and ozone treatments are carried out alternatingly.

8. Process according to one of the claims 5 to 7, characterized by the fact that the process is carried out at least during part of the steam treatment time in the presence of ozone.

9. Process according to one of the claims 5 to 8, characterized by the fact that the ozone as saturated aqueous ozone solution and/or as a gas is brought into contact with the waste to be sterilized.

10. Process according to claim 9, characterized by the fact that the thermal steam treatment is carried out during its entire duration in an ozonic atmosphere.

11. Process according to claim 10, characterized by the fact that at least during the heating phase ozone is injected in a quantity sufficient as to maintain an ozonic atmosphere.

12. Process according to claim 10, characterized by the fact that ozone in the form of an aqueous solution is sprayed into the reaction chamber during the entire process time.

13. Process according to one of the claims 5 to 12, characterized by the fact that the sterilization with ozone is carried out in an atmosphere of reduced oxygen concentration compared with air.

14. Process according to claim 13, characterized by the fact that the sterilization with ozone is carried out in the same atmosphere as the steam treatment.

15. Process according to one of the preceding claims, characterized by the fact that the atmosphere in which the steam treatment and/or ozone treatment phases are carried out is one with an oxygen concentration of less than 15 vol. %.

16. Process according to claim 15, characterized by the fact that the oxygen concentration of the atmosphere is 10 vol. % or less.

17. Process according to one of the preceding claims, characterized by the fact that the steam treatment and/or ozone treatment phases are carried out under normal pressure or hyperpressure.

18. Process according to one of the preceding claims, characterized by the following successive process phases:
a) introduction of one or several containers which contain the waste to be sterilized into a chamber provided with a microwave heater and an exhaust pipe,
b) reduction of the oxygen concentration in the chamber to less than 10 vol.%, c) injection of water or an aqueous ozone solution into the waste container and/or the
interior of the chamber,
d) switching on of the microwave heater and heating of the atmosphere to a sufficient temperature so as to generate steam at normal pressure or at hyperpressure of up to 0.2 MPa (2 bar), and
e) cooling phase.

19. Process according to claim 18, characterized by the fact that the reduction of the oxygen concentration in process phase b) is carried out by injecting an inert gas, preferably nitrogen, into the opening of the waste container and/or into the reaction chamber and/or through bringing the chamber atmosphere into contact with or leading it through a molecular sieve and/or injecting oxygen-reduced air produced with external molecular sieves into the chamber.

20. Process according to claim 18 or 19, characterized by the fact that in process phase c) water or a saturated aqueous ozone solution is injected in a quantity of 5 to 20 l per 100 kg of waste.

21. Process according to one of the claims 18 to 20, characterized by the fact that the chamber is heated at least by one, preferably by several microwave heaters located underneath or on the sides of the waste container.

22. Process according to one of the claims 18 to 21, characterized by the fact that one entire process cycle takes 5 to 30 minutes.

23. Process according to one of the claims 18 to 22, characterized by the fact that during the entire heating phase an aqueous ozone solution or ozone gas is injected.

24. Process according to claim 23, characterized by the fact that the aqueous ozone solution or the ozone gas continues to be injected during the cooling phase e) after the heating phase is terminated.

25. Process according to one of the claims 18 to 24, characterized by the fact that during the heating phase d) the lid of the waste container is cooled, preferably rinsed with nitrogen.

26. Process according to one of the claims 18 to 25, characterized by the fact that during the cooling phase e) the lid of the chamber and/or of the waste container is preferably cooled by rinsing it with nitrogen.

27. Apparatus for carrying out the process according to one of the claims 1 to 26, consisting of a chamber (17) provided with a vapor exit pipe (6), a microwave heating system (21) for heating up the interior of the chamber (22), and a device (1) for injection of an inert gas and/or a molecular sieve to reduce the oxygen concentration in the chamber atmosphere.

28. Apparatus according to claim 27, characterized by the fact that the lid (23) of the chamber is double-walled so as to let pass a coolant, and provided with in- and outlets (24) for the coolant.

29. Apparatus according to one of the claims 27 and 28, characterized by the fact that it is equipped with supply lines for water or an aqueous ozone solution and/or an inert gaseous coolant, said supply lines leading into the chamber (22) and/or into the waste containers (18) placed in the chamber (22).

30. Apparatus according to one of the claims 27 to 29, characterized by the fact that it is equipped with one or several of the following devices:
a) a device for spraying water or an aqueous ozone solution into the chamber and/or into a waste container in the chamber at the start of a process cycle,
b) an automatic fire control system(5) connected with a sprinkler (7),
c) automatic devices for measuring and controlling temperature (8), pressure (9), oxygen concentration (4) and/or degree of humidity (10) in the chamber.

31. Apparatus according to one of the claims 27 to 30, characterized by the fact that it is equipped with a device for direct or indirect cooling, i.e. for condensation of the vapor emitted from the vapor exit pipe.

32. Apparatus according to claim 31, characterized by the fact that the cooling device for condensation of the vapor is immediately followed by a device for chemical sterilization and/or for sterilization by UV radiation of the condensed vapor.

## Revendications

1. Procédé de stérilisation de déchets médicaux toxiques par stérilisation thermique avec de la vapeur d'eau, caractérisé en ce qu'il consiste à mettre les déchets, dans une atmosphère d'une teneur réduite d'oxygène par rapport à l'air, en contact direct avec la vapeur.

2. Procédé selon la revendication 1, caractérisé en ce qu'on opère sous exclusion partielle ou complète d'oxygène.

3. Procédé selon l'une des revendications 1 ou 2, caractérisé en ce qu'il consiste à chauffer de l'eau au moyen d'un générateur de micro-ondes pour obtenir de la vapeur.

4. Procédé selon la revendication 3, caractérisé en ce qu'il consiste à introduire l'eau et les déchets dans la même chambre que l'on soumet à radiation par micro-ondes.

5. Procédé selon l'une des revendications 1 à 4, caractérisé en ce qu'il consiste à effectuer une stérilisation à ozone en plus de la stérilisation thermique à vapeur.

6. Procédé selon la revendication 5, caractérisé en ce que la stérilisation à vapeur et la stérilisation à ozone sont effectuées dans un ordre arbitraire, soit l'une après l'autre, soit simultanément, soit partiellement coïncidant.

7. Procédé selon l'une des revendications 5 ou 6, caractérisé en ce qu'un ou plusieurs traitements à vapeur et traitements à ozone sont effectués de manière alternante.

8. Procédé selon l'une des revendications 5 à 7, caractérisé en ce qu'il est réalisé au moins pendant une partie de la durée du traitement à vapeur en présence d'ozone.

9. Procédé selon l'une des revendications 5 à 8, caractérisé en ce qu'il consiste à mettre l'ozone en tant que solution aqueuse saturée d'ozone et/ou en tant que gaz en contact avec les déchets à stériliser.

10. Procédé selon la revendication 9, caractérisé en ce que le traitement thermique à vapeur est réalisé pendant toute sa durée dans une atmosphère ozonisée.

11. Procédé selon la revendication 10, caractérisé en ce qu'il consiste à injecter, au moins pendant la phase de chauffage, une quantité suffisante d'ozone pour maintenir une atmosphère ozonisée.

12. Procédé selon la revendication 10, caractérisé en ce que l'atomisation de l'ozone, sous forme d'une solution aqueuse, s'effectue pendant toute la durée du procédé.

13. Procédé selon l'une des revendications 5 à 12, caractérisé en ce que la stérilisation à ozone est réalisée dans une atmosphère d'une teneur réduite d' oxygène par rapport à l'air.

14. Procédé selon la revendication 13, caractérisé en ce que la stérilisation à ozone se réalise dans la même atmosphère que celle du traitement à vapeur.

15. Procédé selon l'une des revendications précédentes, caractérisé en ce que l'atmosphère dans laquelle les phases du traitement à vapeur et/ou du traitement à ozone sont effectuées possède une teneur d'oxygène inférieure à 15 % vol.

16. Procédé selon la revendication 15, caractérisé en ce que la teneur d'oxygène de l'atmosphère est égale ou inférieure à 10 % vol.

17. Procédé selon l'une des revendications précédentes, caractérisé en ce que les phases du traitement à vapeur et/ou du traitement à ozone sont réalisées sous pression normale ou surpression.

18. Procédé selon l'une des revendications précédentes, caractérisé par les phases successives qui suivent:
a) introduction d'un ou de plusieurs récipients contenant les déchets à stériliser dans une chambre comportant un générateur de micro-ondes et un tuyau d'évacuation,
b) réduction de la teneur d'oxygène à l'intérieur de la chambre à une valeur inférieure à 10 % vol.,
c) injection d'eau ou d'une solution aqueuse d'ozone dans le récipient et/ou dans l'intérieur de la chambre,
d) allumer le générateur de micro-ondes et chauffage de l'atmosphère à une température suffisante pour la génération de vapeur, sous pression normale ou surpression d'une valeur jusqu'à 0,2 MPa (2 bar), et
e) refroidissement.

19. Procédé selon la revendication 18, caractérisé en ce que la réduction de la teneur d'oxygène dans la phase b) du procédé est effectuée en injectant un gaz inerte (de préférence de l'azote) dans un récipient muni d'un orifice et/ou dans la chambre, et/ou en mettant en contact l'atmosphère dans la chambre avec un tamis moléculaire ou en le conduisant à travers un tel tamis, et/ou en injectant dans la chambre de l'air d'une teneur réduite d'oxygène produit au moyen de tamis moléculaires externes.

20. Procédé selon l'une des revendications 18 ou 19, caractérisé en ce que, dans la phase c), il consiste à injecter de l'eau ou une solution aqueuse saturée d'ozone dans une quantité comprise entre 5 et 20 litres par 100 kg de déchets.

21. Procédé selon l'une des revendications 18 à 20, caractérisé en ce qu'il consiste à chauffer la chambre par au moins un, mais de préférence plusieurs générateurs de micro-ondes installés en-dessous ou aux cotés du récipient.

22. Procédé selon l'une des revendications 18 à 21, caractérisé en ce que la durée du procédé a une longueur comprise entre 5 et 30 minutes.

23. Procédé selon l'une des revendications 18 à 22, caractérisé en ce que pendant toute la durée de la phase de chauffage on injecte une solution aqueuse d'ozone ou du gaz ozonisé.

24. Procédé selon la revendication 23, caractérisé en ce que l' injection de la solution aqueuse d'ozone ou du gaz ozonisé est continuée pendant la phase e) de refroidissement suivant la phase d) de chauffage.

25. Procédé selon l'une des revendications 18 à 24, caractérisé en ce qu'on refroidit le couvercle du récipient pendant la phase d) de chauffage, de préférence en le rinçant avec de l'azote.

26. Procédé selon l'une des revendications 18 à 25, caractérisé en ce qu'on refroidit le couvercle de la chambre et/ou du récipient pendant la phase e) du refroidissement en le rinçant avec de l'azote.

27. Dispositif pour la mise en oeuvre du procédé selon l'une des revendications 1 à 26, caractérisé en ce qu'il comprend une chambre (17) munie d'un conduit d'évacuation de vapeur (6), d'un système de chauffage à micro-ondes (21) pour chauffer l'intérieur de la chambre (22), et d'un dispositif (1) pour l'injection d'un gaz inerte et/ou d'un tamis moléculaire pour la réduction de la teneur d'oxygène dans l'atmosphère de la chambre.

28. Dispositif selon la revendication 27, caractérisé en ce que le couvercle (23) de la chambre est équipé de parois doubles, pour contenir le réfrigérant, et de conduits d'amenée et d'évacuation (24) du réfrigérant.

29. Dispositif selon l'une des revendications 27 et 28, caractérisé en ce qu'il est équipé de conduits d'eau ou d'une solution aqueuse d'ozone et/ou d'un réfrigérant inerte gazeux débouchant dans la chambre (22) et/ou dans le récipient (18) introduit dans la chambre (22).

30. Dispositif selon l'une des revendications 27 à 29, caractérisé en ce qu'il est équipé d'un ou plusieurs dispositifs suivants :
a) un dispositif pour vaporiser l'eau ou une solution aqueuse d'ozone dans la chambre et/ou dans un récipient introduit dans la chambre lors du début d'un cycle du procédé,
b) un système automatique de protection d'incendie (5) qui est relié à un système d'arrosage (7),
c) des dispositifs automatiques pour mesurer et réguler la température (8), la pression (9), la teneur d'oxygène (4) et/ou le degrée d'humidité (10) dans la chambre.

31. Dispositif selon l'une des revendications 27 à 30, caractérisé en ce qu'il est équipé d'un dispositif de refroidissement direct ou indirect pour faire condenser les vapeurs évacuées à travers le conduit d'évacuation de vapeur (2).

32. Dispositif selon la revendication 31, caractérisé en ce que le dispositif de refroidissement pour la condensation des vapeurs évacuées est immédiatement suivi d'un dispositif de stérilisation chimique et/ou de stérilisation par radiation ultraviolette des vapeurs condensées.
